# EUROPEAN PATENT SPECIFICATION

(11) **EP 1 373 226 B1**
(45) Date of publication and mention of the grant of the patent: **04.07.2007**
(21) Application number: 01934298.9
(22) Date of filing: 30.03.2001
(51) Int. Cl.: C07D 241/12, B01J 23/652, B01J 23/60

(54) **PROCESS FOR THE ENHANCEMENT OF CYCLE LIFE OF A ZINC-CHROMIUM BASED CATALYST IN THE SYNTHESIS OF 2-METHYLPYRAZINE**
VERFAHREN ZUR ERHÖHUNG DER LEBENSDAUER EINES AUF ZINK-CHROM BASIERENDEN KATALYSATORS BEI DER SYNTHESE VON 2-METHYLPYRAZIN
PROCEDE SERVANT A AUGMENTER LA DUREE DE VIE D'UN CATALYSEUR A BASE DE ZINC-CHROME DANS LA SYNTHESE DE 2-METHYLPYRAZINE

(43) Date of publication of application: 02.01.2004
(73) Proprietor: COUNCIL OF SCIENTIFIC AND INDUSTRIAL RESEARCH, New Delhi 100 001 (IN)
(72) Inventor: POTHARAJU SEETHARAMANJANEYA, Sai Prasad, Hyderabad 500007, Andhra Pradesh (IN); RAGHAVAN, Kondapuram Vijaya, Hyderabad 500007, Andhra Pradesh (IN); RAO, Panja Kanta, Hyderabad 500007, Andhra Pradesh (IN); KULKARNI, Shivanand Janardan, Hyderabad 500007, Andhra Pradesh (IN); RAO, Katabathini Narasimha, Hyderabad 500007, Andhra Pradesh (IN); GAPINATH, Rajesh, Hyderabad 500007, Andhra Pradesh (IN); FARSINAVIS, Suresh, Hyderabad 500007, Andhra Pradesh (IN); MESHRAM, Harshadas Mitaram, Hyderabad 500007, Andhra Pradesh (IN)
(74) Representative: Geary, Stephen
(86) International application number: PCT/IN2001/000069
(87) International publication number: WO 2002/079171

(56) References cited:
- WO-A-88/00189
- W. GERHARTZ (EDITOR): "Ullmann's encyclopedia of industrial chemistry, fifth edition, Vol. A5" 1986 , VCH , WEINHEIM XP002182488 page 322-324; page 323, item 2.4.3, first two paragraphs
- G. ERTL, H. KNÖZINGER, J. WEITKAMP (EDITORS): "Handbook of heterogeneous catalysis, Vol.3" 1997 , VCH , WEINHEIM XP002182489 page 1275-76 page 1275, item 7.6
- KULKARNI, S. J. ET AL: "Intermolecular and intramolecular cyclization over modified ZSM-5 and chromite catalysts to synthesize 2-methyl pyrazine and piperazine" INDIAN J. CHEM., SECT. A: INORG., BIO-INORG., PHYS., THEOR. ANAL. CHEM. (1993), 32A(1), 28-32 , XP001021056
- FORNI, LUCIO ET AL: "TPD-TPR-MS mechanistic study of the synthesis of 2-methylpyrazine over palladized zinc-chromium oxide" J. CATAL. (1991), 130(2), 403-10 , XP001018236

## Description

### Field of the invention

The present invention relates to a process for the enhancement of the cycle life of zinc chromium based catalyst in the synthesis of 2-methylpyrazine. More particularly, the present invention relates to an improved process for the enhancement of the cycle-life of the catalyst used in the synthesis of 2-methylpyrazine.

### Background of the invention

2-methylpyrazine is used as an intermediate in the production of pyrazinamide, an anti-tubercular drug. It is known to react ethylenediamine and propyleneglycol on a zinc-based catalyst by a vapour phase process to produce 2-methylpyrazine. 2-methylpyrazine, in turn, is converted to 2-cyanopyrazine by catalytic ammoxidation of 2-methylpyrazine using ammonia and oxygen or air. In the last step, 2-cyanopyrazine is hydrolyzed to 2-amidopyrazine, which is popularly called as pyrazinamide.

The duration of time when the catalyst exhibits continuous steady-state activity/selectivity before it starts showing unacceptable activity and selectivity and requires regeneration is normally defined as its cycle-life. After regeneration, the catalyst regains its activity/selectivity either fully or partially and is used for the next cycle. After a few regenerations, the catalyst gets deactivated by exhibiting unacceptable activity/selectivity or both even after regeneration and needs to be replaced with a fresh catalyst. The total time for which the catalyst is put into operation before discharge is called the total life or simply the life of the catalyst. Longer cycle-life and thus longer catalyst life are preferred for catalysts operating in fixed bed reactors. The commercial process becomes economical when the catalyst not only offers high activity and high selectivity towards the required product but also possesses sufficient cycle-life and thus, the total life.

Reference is made to the following zinc-based catalysts which were reported in the prior art for the preparation of 2-methylpyrazine wherein (i) Zinc catalyst promoted with one or more of cobalt, nickel, iron, chromium and aluminum (British Patent 1565117). (ii) ZnCr₂O₄-incorporated ZnO with Pd as promoter (Journal of Applied Catalysis Vol. 29, 1987; Journal of Catalysis, Vol. 111, 1988 and Journal of Chemical Society Transactions Vol. 84(7), 1988; Indian Patent Application No. 280/DEL/92 and Indian Patent Application No. 281/DEL/92).(iii) Catalyst with zinc and chromium in a molar ratio of 1:3 (Journal of Catalysis, Vol. 111, 1988; Catalysis, Vol.3, Rheinhold Publications, New York, Page 349, 1955; Catalytic Manufacture, Dekker, New York, 1983) (iv) Zinc-chromium based catalyst with non-metallic sulphate or bisulphate as promoter (Indian Patent No.176919, 1993)

The drawbacks of the prior art are as follows: The catalyst referred to above in item (i) has a poor cycle life, though the 2-methylpyrazine yields are promising. Zinc based catalysts promoted with Ce, Mg etc., though offer high initial activity, their life times are of the order of 25 hours. Catalyst reported by Forni et al, cited in item (ii) above, has the first cycle life of about 150 hours. Subsequent second and third cycle lives after regenerations were lower compared to the first cycle. The total catalyst life after three regenerations (with the catalyst giving a maximum of 50% conversion), was reported to be 450 hours. Non-metallic sulphate or bisulphate promoted zinc-based catalysts reported in item (iv) referred above also were reported to be tested for 144 hours with acceptable conversions and yields. Thus, the catalysts reported in the prior art possess their cycle lives less than 150 hours.

### Objects of the invention

Accordingly the main object of the present invention is to provide a process for the enhancement of the cycle-life of the catalyst used in the synthesis of 2-methylpyrazine from ethylenediamine and propyleneglycol by a vapour phase catalytic process which obviates the drawbacks as detailed above.

Another object of the present invention is to provide the operating conditions wherein the conversions with respect to ethylenediamine and propyleneglycol are in the order of 100%.

Yet another object of this invention is to provide the operating conditions wherein the yield of 2-methylpyrazine is obtained in the range of 70-80%.

Yet another object of the present invention is to provide the operating conditions wherein the product of the reaction contains pyrazine as the main byproduct and its composition in the product is less than 10-12%.

Still another object of this invention is to provide a method for the regeneration of the catalyst after first cycle life such that the regenerated catalyst again gives ethylenediamine and propyleneglycol conversions and 2-methylpyrazine and pyrazine selectivities similar to those obtained on the fresh catalyst.

### Brief description of the accompanying drawings

Figure 1 is a time on stream analysis of fresh catalyst under step-wise temperature increase conditions.
Figure 2 is a time on stream analysis of regenerated catalyst under step-wise temperature increase conditions.
Figure 3 is a time on stream analysis of fresh catalyst under isothermal conditions.
Figure 4 is a time on stream analysis of the catalyst after second regeneration under step-wise temperature conditions.

### Summary of the invention

Accordingly the present invention provides a process for the synthesis of 2-methylpyrazine, said process comprising reacting ethylenediamine and propyleneglycol at a molar ratio of 1 - 1.5:1 in a fixed bed reactor over a zinc-chromium based catalyst at a temperature in the range of 300 - 409°C, characterised in that an enhancement in cycle life of the zinc-chromium based catalyst is obtained by increasing the temperature of the reaction step-wise dependent on the conversion of the reactant stream to the product stream,with the temperature being increased when the level of pyrazine in the product mixture reaches a maximum level of 10-12%, from an initial temperature of 300 to 350°C until either a final temperature of 380 to 409°C is reached or the level of pyrazine exceeds the maximum level, and in that the weight hourly spaced velocity (WHSV) is 0.15 - 1.00 per hour.

In one embodiment of the invention, the step-wise temperature increase is carried out starting at temperatures in the range 300 - 340°C dependent on the conversion rate of the reactant stream.

In another embodiment of the invention, the conversion of ethylenediamine and propyleneglycol is about 100%.

In a further embodiment of the invention, the yield of 2-methylpyrazine is in the range of 70 - 80% and pyrazine levels in the range of 2 - 12%.

In yet another embodiment of the invention, the catalyst after use is subject to at least one regeneration, said regeneration comprising washing the used catalyst in steam, calcining in air at 400°C till no carbon dioxide is observed in the effluent and reducing in hydrogen at 400°C for 4 hours.

### Detailed description of the invention

The cycle life of the catalyst can be enhanced to 500 hours before regeneration. The catalyst after the first regeneration give another cycle life of 500 hours and after the second regeneration gives yet another cycle life of 500 hours and is found to be still active.

Zinc-chromium catalysts are prepared using the commercial Zn-Cr catalyst (Engelhard-0312 T) as the base material. Aqueous solution of palladium sulphate is impregnated on the base material such that the composition of the palladium sulphate is in the range of 0.5 - 5.0% by weight, preferentially in the range of 1 - 3%. The impregnated catalyst is subjected to drying in air at a temperature in the range of 100 - 150°C.

The catalyst can be used advantageously in the process for the production of 2-methylpyrazine from ethylenediamine and propyleneglycol. The catalyst prepared by the procedure described herein was loaded into a fixed bed reactor and preheated with a gas mixture of hydrogen and nitrogen or hydrogen alone, at a temperature of 300 - 500°C, preferentially in the range of 300 - 400°C, for a period of 4 - 8 hours.

The feed to the reactor can be made by mixing aqueous solutions of ethylenediamine and propyleneglycol in the molar ratio of 3:1, preferentially in the ratio 2:1 and more preferentially 1.2:1. The water content in the feed mixture is 30-80% by volume. The water content can be reduced to 30-60% by volume. Pure nitrogen is optionally fed into the reactor. The liquid feed is vaporized in a preheater and passed into the reactor at a weight hourly space velocity (WHSV) of 0.25 - 1.00 per hour or preferentially in the range of 0.25 - 0.75 per hour.

The catalyst bed is initially maintained at a temperature of 300 - 350°C and the reaction is carried out. The product is collected by cooling the condensables and analyzing them on a gas chromatograph. The reaction is continued at the same temperature as long as the yield of 2-methylpyrazine and the main by-product, pyrazine levels are in the acceptable limits. As the reaction proceeds, the pyrazine levels in the product mixture increase and touch the prescribed maximum limit. At this juncture the reaction temperature is raised by 10 - 50°C and more preferentially in the range of 10 - 30°C. The procedure described above is continued until the reactor temperature reaches 380 - 409°C or the pyrazine levels exceed beyond the set limit.

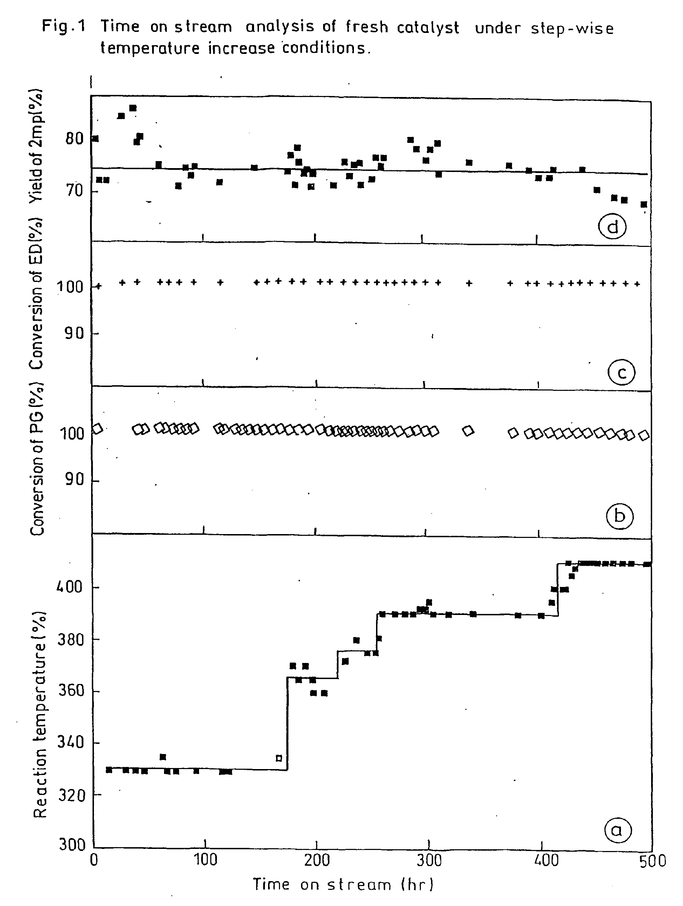

It is observed that by increasing the reaction temperature in steps or stages as described above, the cycle-life of the catalyst can be extended to about 500 hours in a single continuous run before regeneration, as illustrated in Figure 1.

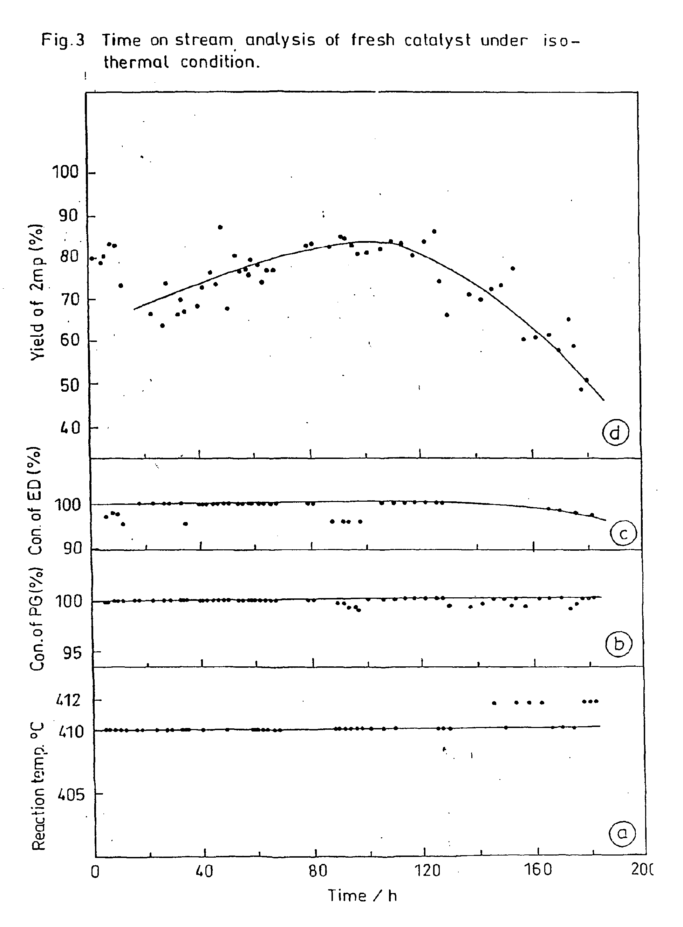

Increasing the cycle-life well beyond 150 hours cannot be achieved if the reaction temperature is maintained continuously at such higher levels more than 409°C as illustrated in Figure 3.

In order to achieve good conversion and selectivity towards 2-methylpyrazine, low initial temperatures are favourable. It is also observed that as long as the catalyst gives good yields of 2-methylpyrazine under the required conversions of ethylenediamine and propyleneglycol, it is advantageous to operate the reactor at low initial reaction temperature, of the order of 300 - 350°C, more preferably in the range of 310 - 340°C.

The catalyst can be regenerated even after working for 500 hours and exhibiting a tendency of reduction in the yields of 2-methylpyrazine or increase in pyrazine levels beyond the acceptable levels. The regeneration procedure involves treating the catalyst first at 400 - 500°C in nitrogen, preferentially in steam for 4 - 6 hours, then oxidizing in air or preferentially in a mixture of oxygen and nitrogen at 400°C for 4 - 6 hours and finally reducing the catalyst in a mixture of hydrogen and nitrogen or hydrogen alone at 300 - 350°C, preferentially at 300 - 400°C for 4 - 6 hours.

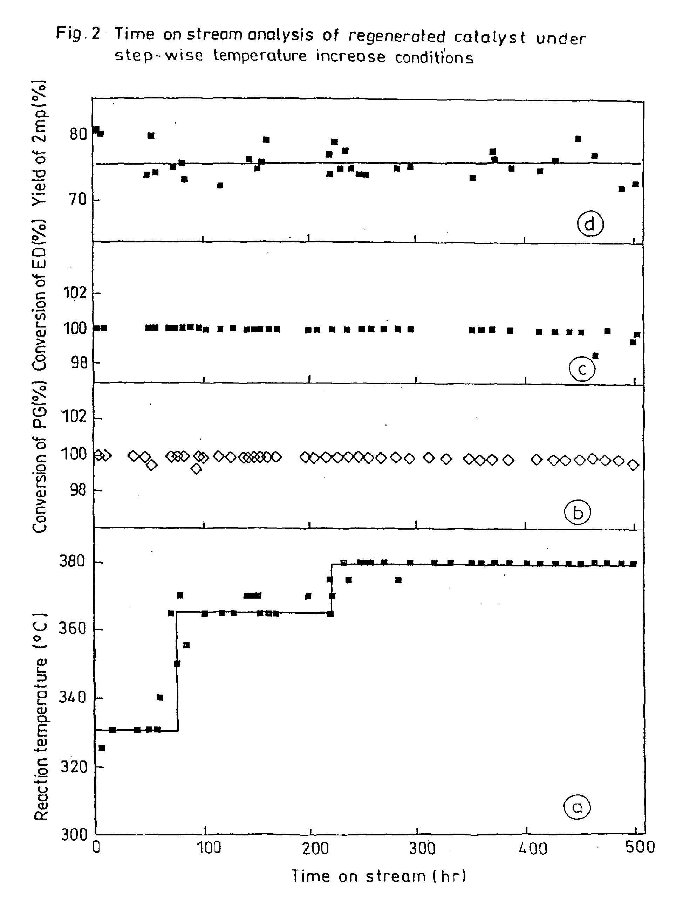

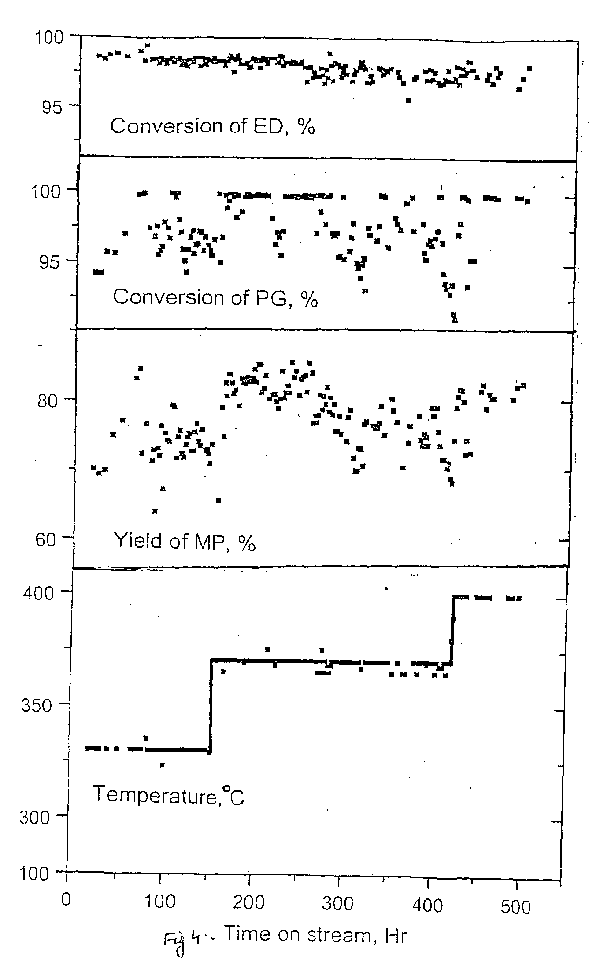

It is observed that the catalyst activity is restored after regeneration. The catalyst, after the first regeneration, is subjected to the reaction temperature again starting at lower temperatures in the range of 300 - 350°C, preferentially in the range of 310 - 340°C and following the same procedure as described in the evaluation of the fresh catalyst. It is observed that the regenerated catalyst is active and selective for the next 500 hours of operation as illustrated in Figure 2. The catalyst after the second regeneration is also found to be active and selective for the next 500 hours of operation as illustrated in Figure 4. Thus, the combined life of the catalyst after the first regeneration itself is 1000 hours and after the second regeneration the life of the catalyst is 1500 hours. However, it cannot be construed that the total life of the catalyst is only 1500 hours. Further regeneration and reactions could enhance the life by several folds. This is an important aspect of the invention since the total life of the best catalyst reported in literature, so far, is of the order of 430 hours, that too, after three regenerations and allowing the conversion to fall to about 50%.

It is observed that for the same catalyst evaluated at temperature more than 400°C, under isothermal conditions, the yield of methylpyrazine drops to less than 70% in about 150 hours as described in Figure 3. This observation strengthens the result of the present invention that isothermal reaction conditions at high temperatures can lead to deactivation of the catalyst after about 150 hours.

The present invention is described with reference to the following examples, which are explained by way of illustration only and should not be construed to limit the scope of the present invention.

### Example 1

About 150 grams of 1.5 wt% palladium sulphate promoted zinc-chromium catalyst was loaded into a fixed bed reactor of 40 mm i.d. provided with a preheater of the same diameter. The liquid feed contained ethylenediamine and propyleneglycol in the molar ratio of 1.1:1. The organic feed was mixed with equal volume of water. The catalyst was slowly heated to and maintained at a reaction temperature of 410°C, in flowing nitrogen after reduction in hydrogen at 400°C for 4 hours. The liquid was introduced into the reactor and the performance of the catalyst was monitored every hour by analysing the product on a gas chromatograph. The catalyst exhibited increasing conversion with increase in time on stream for sometime and later started decreasing. The cycle life of the catalyst as evident from Figure 3 is about 150 hours.

### Example 2

150 grams of another fraction of the fresh catalyst, as used in Example 1, was loaded into the reactor and the same activation procedure was carried out as described in Example 1. After reduction at 400°C for 4 hours the catalyst was cooled to the reaction temperature of 320°C in nitrogen flow and the reaction continued with the liquid feed. The performance of the catalyst was monitored every hour. As long as the yield of 2-methylpyrazine was in the rante of 70 - 80% and the pyrazine levels in the reaction effluent was below 10 - 12%, the reaction was continued. As the pyrazine level crossed beyond the set value, the reaction temperature was increased in steps and the procedure continued. The performance of the catalyst is depicted in Figure 1.

### Example 3

After testing the cycle life of the fresh catalyst for 500 hours, as described in Example 2, the reaction was stopped for regenerating the catalyst. The catalyst was first treated in steam at 400°C for 4 hours, calcined in air at the same temperature till the reactor effluent showed no indication of carbon dioxide formation and then it was reduced at the same temperature in hydrogen for 4 hours. The reaction was continued again at 320°C following the stepwise temperature increasing policy. The performance of the regenerated catalyst is depicted in Figure 2. The catalyst exceeds 500 hours of operation even at 380°C with required performance parameters giving further scope of increased cycle life.

### Example 4

After testing the cycle life of the first time regenerated catalyst for 500 hours, as described in Example 2, the reaction was stopped for the second regeneration. The catalyst was first treated in steam at 400°C for 4 hours, calcined in air at the same temperature till the reactor effluent showed no indication of carbon dioxide formation and then it was reduced at the same temperature in hydrogen for 4 hours. The reaction was continued again at 320°C following stepwise increase in temperature. The performance of the second time regenerated catalyst is depicted in Figure 4. The catalyst gave another 500 hours of operation with the required performance parameters giving further scope for increased cycle life.

### The main advantages of the present invention are:

1. The reactor can be operated at lower temperatures, 320 - 330°C instead of 380 - 400°C at the beginning of the reaction, still getting the required performance like 100% conversion of the reactants and getting required yield of 2-methylpyrazine.
2. The cycle life of the catalyst can be increased to 500 hours by operating the reactor adopting the stepwise temperature increase method. Isothermal operation at high reaction temperature is reported to give a cycle life of less than 150 hours only.
3. The catalyst after the first cycle can be regenerated by the procedure described in this invention.
4. The regenerated catalyst, after the first cycle-life evaluation, performs better than the fresh catalyst in terms of conversion and selectivity.
5. Though the life of the catalyst is established as 1500 hours, its actual life can be further extended with additional experimentation since the catalyst is not deactivated.

## Claims

1. A process for the synthesis of 2-methylpyrazine, said process comprising reacting ethylenediamine and propyleneglycol at a molar ratio of 1 - 1.5:1 in a fixed bed reactor over a zinc-chromium based catalyst at a temperature in the range of 300 - 409°C, **characterised in that** an enhancement in cycle life of the zinc-chromium based catalyst is obtained by increasing the temperature of the reaction step-wise dependent on the conversion of the reactant stream to the product stream,with the temperature being increased when the level of pyrazine in the product mixture reaches a maximum level of 10-12%, from an initial temperature of 300 to 350°C until either a final temperature of 380 to 409°C is reached or the level of pyrazine exceeds the maximum level, and **in that** the weight hourly spaced velocity (WHSV) is 0.15 - 1.00 per hour.

2. A process as claimed in Claim 1, **characterised in that** the step-wise temperature increase is carried out starting at temperatures in the range of 300 - 340°C dependent on the conversion rate of the reaction stream.

3. A process as claimed in Claim 1, **characterised in that** the conversion of ethylenediamine and propyleneglycol is about 100%.

4. A process as claimed in Claim 1, **characterised in that** the yield of 2-methylpyrazine is in the range of 70 - 80% and pyrazine levels in the range of 2 - 12%.

5. A process as claimed in Claim 1, **characterised in that** the catalyst after use is subject to at least one regeneration said regeneration comprising washing the used catalyst in stream, calcining in air at 400°C till no carbon dioxide is observed in the effluent and reducing in hydrogen at 400°C for 4 hours.

## Patentansprüche

1. Verfahren zur Synthese von 2-Methylpyrazin, wobei das Verfahren umfasst: Reagieren von Ethylendiamin und Propylenglykol in einem Molverhältnis von 1 bis 1,5:1 in einem Festbettreaktor über einem Katalysator auf Zink-Chrom-Basis bei einer Temperatur im Bereich zwischen 300 und 409°C, **dadurch gekennzeichnet, dass** die Zyklenlebensdauer des Katalysators auf Zink-Chrom-Basis verbessert wird, indem die Reaktionstemperatur in Abhängigkeit der Umsetzung des Reaktantenstroms in den Produktstrom schrittweise erhöht wird, wobei, wenn der Pyrazinpegel in der Produktmischung einen maximalen Pegel von 10-12% erreicht hat, die Temperatur von einer Anfangstemperatur von 300 bis 350°C so lange erhöht wird, bis entweder eine Endtemperatur von 380 bis 409°C erreicht ist oder der Pyrazinpegel den maximalen Pegel überschreitet, und dass die gewichtsbezogene stündliche Raumgeschwindigkeit (weight hourly space velocity = WHSV) zwischen 0,15 und 1,00 pro Stunde beträgt.

2. Verfahren nach Anspruch 1, **dadurch gekennzeichnet, dass** der schrittweise Temperaturanstieg ausgeführt wird beginnend bei Temperaturen im Bereich von 300 bis 340°C in Abhängigkeit der Umsetzungsrate des Reaktantenstroms.

3. Verfahren nach Anspruch 1, **dadurch gekennzeichnet, dass** die Umsetzung von Ethylendiamin und Propylenglykol ca. 100% beträgt.

4. Verfahren nach Anspruch 1, **dadurch gekennzeichnet, dass** der Ertrag von 2-Methylpyrazin im Bereich zwischen 70 und 80% liegt und die Pyrazinpegel im Bereich zwischen 2 und 12% liegen.

5. Verfahren nach Anspruch 1, **dadurch gekennzeichnet, dass** der Katalysator nach der Verwendung mindestens einer Regeneration unterzogen wird, wobei die Regeneration das Waschen des verwendeten Katalysators in dem Strom, Kalzinieren in Luft bei 400°C, bis kein Kohlenstoffdioxid in dem Abwasser mehr beobachtet wird, und Reduzieren in Wasserstoff bei 400°C über 4 Stunden umfasst.

## Revendications

1. Procédé de synthèse de la 2-méthylpyrazine, ledit procédé comprenant la réaction de l'éthylènediamine et du propylèneglycol à un rapport molaire de 1-1,5 :1 dans un réacteur à lit fixe sur un catalyseur à base de zinc-chrome à une température entre 300 et 409°C, **caractérisé en ce qu'**une augmentation dans la durée de vie du catalyseur à base de zinc-chrome est obtenue en augmentant la température de la réaction par étape en dépendance de la conversion du courant de réactifs en courant de produits, la température étant augmentée lorsque le niveau de pyrazine dans le mélange de produits atteint un niveau maximum de 10-12%, à partir de la température initiale de 300 à 350°C jusqu'à ce que, soit une température finale de 380 à 409°C soit atteinte, soit le niveau de pyrazine excède le niveau maximum, et **en ce que** la vitesse spatiale horaire massique (WHSV) est de 0,15-1,00 par heure.

2. Procédé selon la revendication 1, **caractérisé en ce que** l'augmentation par étape de la température est accomplie en démarrant à des températures entre 300 et 340°C en dépendance du taux de conversion du courant de réactifs.

3. Procédé selon la revendication 1, **caractérisé en ce que** la conversion d'éthylènediamine et de propylèneglycol est d'environ 100%.

4. Procédé selon la revendication 1, **caractérisé en ce que** le rendement de la 2-méthylpyrazine est dans la plage 70-80% et les niveaux de pyrazine dans la plage 2-12%.

5. Procédé selon la revendication 1, **caractérisé en ce que** le catalyseur après utilisation est soumis à au moins une régénération, ladite régénération comprenant un lavage du catalyseur utilisé dans le courant, une calcination à l'air à 400°C jusqu'à ce qu'aucun dioxyde de carbone ne soit observé dans l'effluent et la réduction en hydrogène à 400°C pendant 4 heures.
